# EUROPEAN PATENT SPECIFICATION

(11) **EP 0 533 703 B1**
(45) Date of publication and mention of the grant of the patent: **15.03.2000**
(21) Application number: 91910204.6
(22) Date of filing: 23.05.1991
(51) Int. Cl.: C07J 31/00, C07J 9/00, C07J 51/00, C07J 7/00, C07J 5/00, A61K 31/56, C07J 3/00, C07J 71/00

(54) **Use of steroids for the inhibition of angiogenesis**
Verwendung von Steroiden zur Inhibierung von Angiogenesis
Utilisation des steroides pour l'inhibition d'angiogenese

(30) Priority: 11.06.1990 US 536894; 06.11.1990 US 609661
(43) Date of publication of application: 31.03.1993
(73) Proprietor: ALCON LABORATORIES, INC., Fort Worth Texas 76134-2099 (US)
(72) Inventor: WILKS, John, William, Kalamazoo, MI 49008 (US); DEKONING, Thomas, Frank, Marcellus, MI 49067 (US); ARISTOFF, Paul, Adrian, Portage, MI 49002 (US)
(74) Representative: Perry, Robert Edward
(86) International application number: US9103459
(87) International publication number: WO9119731

(56) References cited:
- EP-A- 0 123 241
- EP-A- 0 156 643
- EP-A- 0 186 948
- EP-A- 0 221 705
- EP-A- 0 268 400
- WO-A-87/01706
- DE-A- 2 649 753
- US-A- 3 067 193
- US-A- 3 067 194
- US-A- 3 158 601
- US-A- 3 300 483
- THE JOURNAL OF ORGANIC CHEMISTRY, vol. 44, no. 9, 27 April 1979, American Chemical Society, V. VANRHEENEN et al.: "New synthesis of cortico steroids from 17-keto steroids: Application and stereochemical study of the unsaturated sulfoxide-sulfenate rearrangement", pages 1582-1584, see page 1582, compound 10
- THE JOURNAL OF MEDICINAL CHEMISTRY, vol. 33, no. 4, April 1990, American Chemical Society, E.J. JACOBSEN et al.: "Novel 21-aminosteroids that inhibit iron-dependent lipid peroxidation and protect against central nervous system trauma", pages 1145-1151, see page 1147, compound 25; page 1150, method A
- STEROIDS, vol. 44, no. 4, October 1984, G. ZOMER et al.: "The synthesis of [1,2,3,4-13C] cortisol", pages 293-300, see page 295, compound 10

## Description

### Field of the Invention

The present invention relates to the treatment of angiogenesis in mammals, using certain angiostatic Δ⁴'⁹⁽¹¹⁾-steroids. These steroids are useful in treating diseases of neovascularization such as cancer, diabetes and arthritis

### Description of the Related Art

Angiogenesis is the development of blood vessels which typically would lead to a vascular bed capable of sustaining viable tissue. Angiogenesis is a necessary process in the establishment of embryonic tissue and development of a viable embryo. Similarly, angiogenesis is a necessary step in the establishment and development of tumor tissue as well as certain inflammatory conditions. The inhibition of angiogenesis would be useful in the control of embryogenesis, inflammatory conditions, and tumor growth, as well as numerous other conditions.

EP-A-0 114 589 describes the use of cortisone, hydrocortisone and 11α-hydrocortisone in combination with heparin in the inhibition of angiogenesis.

The angiogensis inhibitory effects of heparin and heparin fragments in combination with cortisone is described in Science 221, 719 (1983). The use of heparin and heparin fragments in combination with hydrocortisone is set forth in the Proceedings of AACR 26, 384 (1985).

Heparin is presently used with inhibitors of angiogenesis, especially angiostatic steroids to treat diseases involving neovascularization, see Biochem. Pharmacol. 34, 905 (1985) and Annals of Surgery 206, 374 (1987). The heparin potentiates the angiogenesis-inhibiting activity of other drugs, for example of collagen biosynthesis inhibitors such as L-azetidine carboxylic acid. The problem with using heparin is that the efficacy of each preparation/batch of heparin differs due to the chemical heterogeneity of the heparin molecules.

Suramin inhibits the binding of fibroblast growth factor to its receptor during in vitro experiments. Fibroblast growth factor is one of a number of known angiogenic growth factors. See, J. Cell Physiol. 132, 143 (1987).

Suramin and 4,4'-bis[[4-(o-hydroxyanilino)-6-(m-sulfoanilino)-s-triazin-2-yl]amino]-2,2'stilbenedisulfonic acid have been reported to possess antitumor activity. See, Gann 61, 569 (1970) and J. Clin. Oncol., 7, 499 (1989).

US Patent 4,599,331 discloses 20-substituted Δ^{1,4}-16-methyl steroids which do not have a Δ⁹⁽¹¹⁾ double bond,which are useful as antiangiogenics when combined with heparin.

US Patent 4,771,042 discloses 21-hydroxy steroids which are useful in the inhibition of angiogenesis involving the co-administration of steroids with heparin or heparin fragments. The compounds of the present invention do not include -CH₂- at C₂₁.

WO87/02672 discloses various C₁₁-functionalized. steroids useful in the inhibition of angiogenesis when combined with heparin.

The Journal of the National Cancer Institute 81, 1346 (1989) discloses that "Suramin also appears to have antiangiogenesis activity ...".

The combination of suramin-type compounds and angiostatic steroids has been reported to treat angiogenesis in a warm blooded mammal; see the Journal of the National Cancer Institute 81, 1346 (1989).

It is known that steroids alone can inhibit angiogenesis, see National Academy of Sciences USA 78, 1176 (1981) [medroxyprogesterone], Cancer Letters 43, 85 (1988) [medroxyprogesterone acetate], International Journal of cancer 35, 549 (1985) [cortisone], JNCI 74, 869 (1985) [cortisone], Cancer Research 47, 5021 (1987) [cortisone acetate] and European Journal of Cancer and Clinical Oncology 23, 93 (1987) [cortisone acetate]. The data reported in these publications are consistent with the clinical practice of using steroids to inhibit tumors.

EP-A-0221705 discloses tetrahydrosteroids which are useful in inhibiting angiogenesis. One such compound is tetrahydrocortisol.

WO-A-89/06536 discloses the use of 17α,21-dihydroxy-16α-methylpregna-4,9(11)-diene-3,20-dione 21-acetate for use in treating angiogenesis.

WO-A-90/15816 discloses the use of steroids for treating angiogenesis. These steroids include 6α-fluoro-17α,21-dihydroxy-16β-methylpregna-4,9(11)-diene-3,20-dione 21-acetate, 6α-fluoro-17α-hydroxy-16α-nethylandrosta-4,9(11)-dien-3-one-17β-carboxylic acid methylester 17-butyrate and 6α-fluoro-17α-hydroxy-16β-nethylandrosta-4,9(11)-dien-3-one-17β-carboxylic acid 17-butyrate.

### SUMMARY OF THE INVENTION

A first aspect of the present invention is the use of a steroid of formula (II) 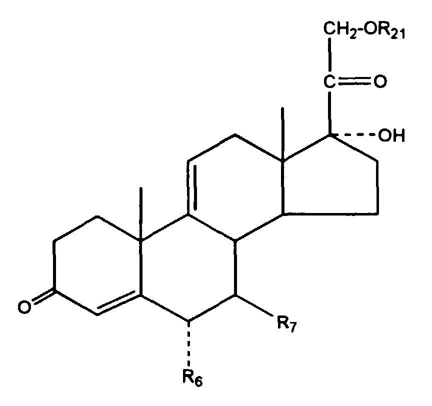 wherein
R₆ is H, F or CH₃;
R₇ is H or CH₃; and
R₂₁ is -CO(C₁₋₁₂ alkyl);
   for the manufacture of a medicament for use in treating, i.e. inhibiting and/or preventing, angiogenesis in a human or other warm-blooded mammal.

A second aspect of the present invention is the use of any of
6α-fluoro-17α,21-dihydroxy-16β-methylpregna-4,9(11)-diene-3,20-dione 17,21-diacetate,
21-bromo-3α,17α-dihydroxy-5β-pregnan-20-one,
17α,21-dihydroxypregna-1,4,9(11)-triene-3,20-dione,
6α-fluoro-17α-hydroxy-16α-methylandrosta-4,9(11)-dien-3-one 17β-carboxylic acid methyl ester 17-butyrate,
17β-carboxy-9β, 11β-epoxy-6α-fluoro-17α-hydroxy-16α-methylandrosta-1,4-dien-3-one 17α-butyrate 17β-methyl ester, and
17β-carboxy-6α-fluoro-17α-hydroxy-16β-methylandrosta-4,9(11)-dien-3-one 17α-butyrate,
in the absence of a suramin-type compound,
for the manufacture of a medicament for use in treating angiogenesis in a human or other warm-blooded mammal.

### DESCRIPTION OF THE INVENTION

Compounds of formula II are known. See, for example, US-A-4975537. They can be readily prepared from steroids well known in the art by methods known to those. skilled in the art.

R₂₁ is preferably COCH₃. The preferred compound of formula II, i.e. 6α-methyl-17α,21-dihydroxypregna-4,9(11)-diene-3,20-dione 21-acetate, is disclosed in WO-A-86/00907.

Compounds for use in the second aspect of this invention are also known. The first three are disclosed in, respectively, US-A-3980778 (column 2, compound 3) and US-A-4018918 (column 10, compound 17); J. Org. Chem. 33:1695 (1968); and US-A-4704358 and US-A-4771042. The fourth compound is also known; see also Example 1, below. The fifth and sixth compounds may be made by methods well known to those skilled in the art.

The steroids to which this invention relates are useful in treating angiogenesis, and especially diseases of neovascularisation. It is preferred that the indication for treatment is selected from solid tumours, diabetes, arthritis, atherosclerosis, neovascularisation of the eye, glaucoma, parasitic diseases, psoriasis, abnormal wound healing processes, hypertrophy following surgery, burns, injury, inhibition of hair growth, inhibition of ovulation and corpus luteum formation, inhibition of implantation and inhibition of embryo development in the uterus. It is more preferred that the neovascular disease is solid tumours, diabetes or arthritis.

The dose of the steroid is from 0.1 to 100 mg/kg/day, preferably from 0.1 to 50 mg/kg/day. The exact dosage and frequency of administration depends on the particular steroid being used, the particular condition being treated, the severity of the condition being treated, the age, weight, general condition of the particular patient, and other medication that the individual may be taking, as is well known to those skilled in the art. These factors can be more accurately determined by measuring the blood level or concentration of the steroid in the patient's blood and/or the patient's response to the particular condition being treated.

For the inhibition of angiogenesis, the steroids of the invention may be combined with each other or with other agents such as suramin (in the first aspect of the invention only), sulfated glycosaminoglycans and sulfated polysaccharides, or effective fragments of these molecules. The preferred glycosaminoglycans include heparin and heparan sulfate. Fragments of heparin or heparan sulfate may also be used if they contain a minimum of six saccharide residues; fragments of heparin and heparan sulfate may be prepared from heparin or heparan sulfate isolated from natural sources, or they may be prepared by chemical synthesis. The steroids of the invention may also be combined with polysaccharides including pentosan polysulphate, cyclodextrins, or other sulfated polysaccharides isolated from natural sources. The preferred polysaccharides are sulfated forms of β-cyclodextrin including β-cyclodextrin tetradecasulfate, pentosan polysulphate, or the polysaccharide-peptidoglycan isolated from Arthrobacter, Journal of Biochemistry 92, 1775 (1982). These polysaccharides may be isolated from natural sources, or prepared by chemical synthesis.

The steroids of the invention may also be used in combination treatments containing compounds which interfere with collagen biosynthesis. Preferred compounds in this group include L-azetidine-2-carboxylic acid, thioproline, and related proline analogues. Also included are other inhibitors of basement membrane collagen synthesis such as 8,9-dihydroxy-7-methylbenzo(b)quinolizinium bromide.

The following Example illustrates the preparation of a steroid for use in this invention.
All temperatures are in degrees Centigrade.
TLC refers to thin-layer chromatography.
MS refers to mass spectrometry expressed as m/e or mass/charge unit. [M + H]⁺ refers to the positive ion of a parent plus hydrogen atom. EI refers to electron impact. CI refers to chemical ionisation. FAB refers to fast atom bombardment.

### EXAMPLE 1

A. Methanol (20 ml) and sodium methoxide (25%, 0.2 ml) are added to 6α-fluoro-17α,21-dihydroxy-16α-methylpregna-4,9(11)-diene-3,20-dione 21-acetate (US Patent 3,291,815, 1.0 g) in methanol. The reaction mixture is neutralized with acetic acid and concentrated to dryness under reduced pressure. The concentrate is distributed between water and chloroform. The organic layer is separated and washed twice with water and dried over anhydrous sodium sulfate. The crude solid is chromatographed over silica gel eluting with ethyl acetate/hexane (35/65). The appropriate fractions are pooled and concentrated to give 6α-fluoro-17α,21-dihydroxy-16α-methylpregna-4,9(11)-diene-3,20-dione, mp 206-207°.
B. THF (26 ml) and periodic acid (0.677 g) in water (10 ml) are added to 611 mg (1.62 mmol) of 6α-fluoro-17,21-dihydroxy-16α-methylpregna-4,9(11)-diene-3,20-dione (Part A, 611 mg). The resulting solution is heated at reflux for 2 hours, then cooled to 25° and concentrated under reduced pressure to a volume of 5 ml. Water (15 ml) is added to the residue and the resulting mixture is extracted with ethyl acetate (2 x 25 ml). The ethyl acetate extracts are combined, dried over anhydrous sodium sulfate, filtered, and concentrated to dryness. The crude material is crystallized from acetone/-hexane to give 6α-fluoro-17α-hydroxy-16α-methylandrosta-4,9(11)-dien-3-one 17β-carboxylic acid, mp 213.8-214°, MS calculated 363.1971, found 363.1962.
C. Acetic anhydride (0.5 ml) and triethylamine (0.3 ml) are added to 6α-fluoro-17α-hydroxy-16α-methylandrosta-4,9(11)-dien-3-one 17β-carboxylic acid (Part B, 300 mg). The resulting mixture is stirred at 20-25° until dissolution occurrs, and then stirred for an additional 40 min. The reaction solution is concentrated to dryness under reduced pressure, and the residue is dissolved in methanol and allowed to sit at 25° for 30 min. Evaporation of the methanol and final drying under high vacuum gives crude 6α-fluoro-17α-hydroxy-16α-methylandrosta-4,9(11)-dien-3-one 17β-carboxylic acid 17-acetate in quantitative yield, TLC R_{f} = 0.05 (ethyl acetate/hexane, 35/65).
D. The crude 17-acetate (Part C) is dissolved in THF (8 ml) and then treated with freshly prepared diazomethane in ether until all of the starting material appeared to have reacted by TLC. The crude product is purified by chromatography over silica gel eluting with ethyl acetate/hexane (25/75). The appropriate fractions are pooled and concentrated to give 6α-fluoro-17α-hydroxy-16α-methylandrosta-4,9(11)-dien-3-one 17β-carboxylic acid methyl ester 17-acetate, TLC R_{f} = 0.6 (ethyl acetate/hexane (35/65); MS calculated 419.2234, found 419.2212.

## Claims

1. Use of a steroid of formula (II) 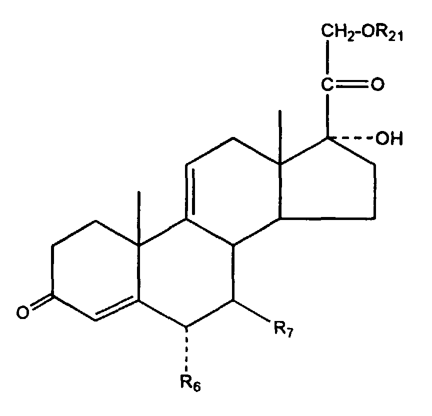 wherein
R₆ is H, F or CH₃;
R₇ is H or CH₃; and
R₂₁ is -CO(C₁₋₁₂ alkyl);
for the manufacture of a medicament for use in treating angiogenesis in a human or other warm-blooded mammal.

2. Use according to claim 1, where the steroid (II) is
6α-methyl-17α,21-dihydroxypregna-4,9(11)-diene-3,20-dione 21-acetate.

3. Use of any of
6α-fluoro-17α,21-dihydroxy-16β-methylpregna-4,9(11)-diene-3,20-dione 17,21-diacetate,
21-bromo-3α,17α-dihydroxy-5β-pregnan-20-one,
17α,21-dihydroxypregna-1,4,9(11)-triene-3,20-dione,
6α-f1uoro-17α-hydroxy-16α-methylandrosta-4,9(11)-dien-3-one 17β-carboxylic acid methyl ester 17-butyrate,
17β-carboxy-9β,11β-epoxy-6α-fluoro-17α-hydroxy-16α-methylandrosta-1,4-dien-3-one 17α-butyrate 17β-methyl ester, and
17β-carboxy-6α-fluoro-17α-hydroxy-16β-methylandrosta-4,9(11)-dien-3-one 17α-butyrate,
in the absence of a suramin-type compound,
for the manufacture of a medicament for use in treating angiogenesis in a human or other warm-blooded mammal.

4. Use according to any of claims 1 to 3, where the medicament is adapted for oral or parenteral administration.

5. Use according to any of claims 1 to 4, for the treatment of any of solid tumours, diabetes, arthritis, atherosclerosis, neovascularisation of the eye, glaucoma, parasitic diseases, psoriasis, abnormal wound-healing processes, hypertrophy following surgery, burns, injury, inhibition of hair growth, inhibition of ovulation and corpus luteum formation, inhibition of implantation and inhibition of embryo development in the uterus.

## Patentansprüche

1. Verwendung eines Steroids der Formel (II) 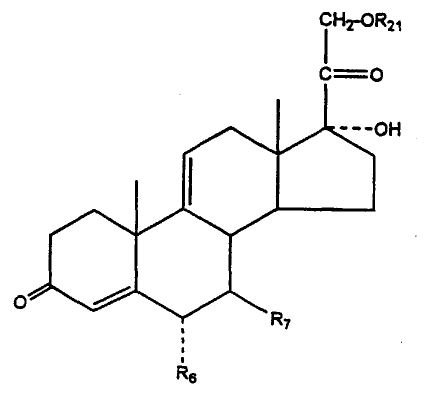 worin
R₆ für H, F oder CH₃ steht;
R₇ für H oder CH₃ steht; und
R₂₁ für -CO(C₁₋₁₂-Alkyl) steht;
für die Herstellung eines Medikaments zur Verwendung bei der Behandlung von Gefäßbildung in einem Menschen oder einem anderen warmblütigen Säuger.

2. Verwendung nach Anspruch 1, bei welcher das Steroid (II) 6α-Methyl-17α,21-dihydroxypregna-4,9(11)-dien-3,20-dion-21-acetat ist.

3. Verwendung von irgendeinem von 6α-Fluor-17α,21-dihydroxy-16β-methylpregna-4,9(11)-dien-3,20-dion-17,21-diacetat, 21-Brom-3α,17α-dihydroxy-5β-pregnan-20-on, 17α,21-Dihydroxypregna-1,4,9(11)-trien-3,20-dion, 6α-Fluor-17α-hydroxy-16α-methylandrosta-4,9(11)-dien-3-on-17β-carbonsäuremethylester-17-butyrat, 17β-Carboxy-9β,11β-epoxy-6α-fluor-17α-hydroxy-16α-methylandrosta-1,4-dien-3-on-17α-butyrat-17β-methylester und 17β-Carboxy-6α-fluor-17α-hydroxy-16β-methylandrosta-4,9(11)-dien-3-on-17α-butyrat in Abwesenheit einer Verbindung vom Suramin-Typ für die Herstellung eines Medikaments zur Verwendung bei der Behandlung von Gefäßbildung in einem Menschen oder einem anderen warmblütigen Säuger.

4. Verwendung nach irgendeinem der Ansprüche 1 bis 3, bei welcher das Medikament für die orale oder parenterale Verabreichung angepaßt ist.

5. Verwendung nach irgendeinem der Ansprüche 1 bis 4 für die Behandlung von irgendeinem von massiven Tumoren, Diabetes, Arthritis, Atherosklerose, Neovaskularisation des Auges, Glaucom, Parasiten-Krankheiten, Psoriasis, abnormalen Wundheilungsprozessen, Hypertrophie im Anschluß an eine Operation, Verbrennungen, Verletzungen, Hemmung des Haarwachstums, Hemmung des Eisprungs und der Corpus luteum-Bildung, Hemmung von Einnistung und Hemmung von Embryo-Entwicklung in der Gebärmutter.

## Revendications

1. Utilisation d'un stéroide de formule (II), 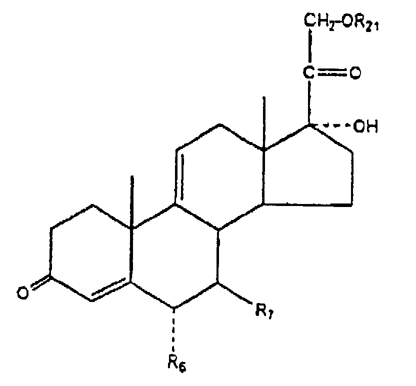
dans laquelle R₆ représente H, F ou CH₃;
R₇ représente H ou CH₃; et
R₂₁ représente -CO(alkyle en C₁₋₁₂);
pour la fabrication d'un médicament à utiliser dans le traitement de l'angiogenèse chez l'homme ou un autre mammifère à sang chaud.

2. Utilisation selon la revendication 1, dans laquelle le stéroide (II) est le 21-acétate de 6α-méthyl-17α,21-dihydroxypregna-4,9(11)-diène-3,20-dione,

3. Utilisation de l'un quelconque parmi
le 17,21-diacétate de 6α-fluoro-17α,21-dihydroxy-16β-méthylpregna-4,9(11)-diène-3,20-dione,
le 21-bromo-3α,17α-dihydroxy-5β-pregnan-20-one,
le17α,21-dihydroxypregna-1,4,9(11)-triène-3,20-dione,
la méthyl ester 17-butyrate d'acide 6α-fluoro-17α-hydroxy-16α-méthylandrosta-4,9(11)-dièn-3-one 17β-carboxylique,
le 17β-méthyl ester 17α-butyrate de 17β-carboxy-9β-11β-époxy-6α-fluoro-17α-hydroxy-16α-méthylandrosta-1,4-dièn-3-one, et
le 17α-butyrate de 17β-carboxy-6α-fluoro-17α-hydroxy-16β-méthylandrosta-4,9 (11)-dièn-3-one,
en l'absence d'un composé de type suramine,
pour la fabrication d'un médicament à utiliser dans le traitement de l'angiogenèse chez l'homme ou un autre mammifère à sang chaud.

4. Utilisation selon l'une quelconque des revendications 1 à 3, dans laquelle le médicament est adapté pour une administration orale ou parentérale.

5. Utilisation selon l'une quelconque des revendications 1 à 4, pour le traitement de l'une quelconque parmi les tumeurs massives, le diabète, l'arthrite, l'athérosclérose, la néovascularisation de l'oeil, le glaucome, dus maladies parasitaires, le psoriasis, des processus anormaux de guérison de blessures, l'hypertrophie suite à de la chirurgie, des brûlures, des blessures, l'inhibition de la croissance des cheveux, l'inhibition de l'ovulation et de la formation du corpus lutcum, l'inhibition de l'implantation et l'inhibition du développement de l'embryon dans l'utérus.
